# EUROPEAN PATENT SPECIFICATION

(11) **EP 1 796 696 B1**
(45) Date of publication and mention of the grant of the patent: **06.04.2011**
(21) Application number: 05815862.7
(22) Date of filing: 30.08.2005
(51) Int. Cl.: A61P 1/04, A61K 36/48, A61K 36/00

(54) **COMPOUNDS, COMPOSITIONS, FORMULATIONS AND PROCESS FOR PREPARATION THEREOF AND METHOD OF TREATMENT AND MANAGEMENT OF ACIDITY AND RELATED DISORDERS**
VERBINDUNGEN, ZUSAMMENSETZUNGEN, FORMULIERUNGEN UND HERSTELLUNGSVERFAHREN DAFÜR, BEHANDLUNGSVERFAHREN, HANDHABUNG VON ÜBERSÄUERUNG UND VERWANDTE ERKRANKUNGEN
COMPOSES, COMPOSITIONS, FORMULATIONS ET LEUR PROCEDE DE PREPARATION ET METHODE DE TRAITEMENT DE L'ACIDITE ET TROUBLES APPARENTES

(30) Priority: 01.09.2004 IN MU09472004
(43) Date of publication of application: 20.06.2007
(60) Divisional application: 10183169.1
(73) Proprietor: Savangikar, Chitra Vasant, Mumbai- 400 018, Maharashtra (IN); Savangikar, Vasant Anantrao, Mumbai- 400 018, Maharashtra (IN)
(72) Inventor: Savangikar, Chitra Vasant, Mumbai- 400 018, Maharashtra (IN); Savangikar, Vasant Anantrao, Mumbai- 400 018, Maharashtra (IN)
(74) Representative: Williams, Paul Edwin
(86) International application number: PCT/IN2005/000289
(87) International publication number: WO 2006/025072

(56) References cited:
- WO-A-99/25197
- FR-M- 4 710
- GB-A- 886 170
- US-A- 3 735 007
- PANDIAN R SUJA ET AL: "Gastroprotective effect of fenugreek seeds (Trigonella foenum graecum) on experimental gastric ulcer in rats" JOURNAL OF ETHNOPHARMACOLOGY, vol. 81, no. 3, August 2002 (2002-08), pages 393-397, XP002366171 ISSN: 0378-8741
- AL-HABORI M ET AL: "ANTIDIABETIC AND HYPOCHOLESTEROLAEMIC EFFECTS OF FENUGREEK" PHYTOTHERAPY RESEARCH, JOHN WILEY & SONS LTD. CHICHESTER, GB, vol. 12, no. 4, 1998, pages 233-242, XP000909006 ISSN: 0951-418X
- SAVANGIKAR V A ET AL: "EDIBLE PROTEIN FROM PARTHENIUM-HYSTEROPHORUS" EXPERIMENTAL AGRICULTURE, vol. 14, no. 1, 1978, pages 93-94, XP009060415 ISSN: 0014-4797
- NEMA R K ET AL: "Soya protein complexes as gastric antacids" INDIAN JOURNAL OF NATURAL PRODUCTS, vol. 12, no. 2, 1996, pages 22-25, XP009060427 ISSN: 0970-129X
- LOZANO PEDRO ET AL: "Food Protein Nutrient Improvement by Protease at Reduced Water Activity" JOURNAL OF FOOD SCIENCE, vol. 59, no. 4, 1994, pages 876-880, XP009060659 ISSN: 0022-1147
- HARJU E ET AL: "THE PROTECTIVE EFFECT OF NUTRIENTS AGAINST STRESS INDUCED GASTRIC ULCERS IN THE RAT" SURGERY GYNECOLOGY AND OBSTETRICS, vol. 165, no. 6, 1987, pages 530-534, XP009060416 ISSN: 0039-6087
- OFFENKRANTZ W G: "Use of fortified defatted dry milk protein in treatment of peptic ulcers." THE AMERICAN JOURNAL OF GASTROENTEROLOGY. OCT 1958, vol. 30, no. 4, October 1958 (1958-10), pages 439-446, XP009060420 ISSN: 0002-9270

## Description

### Technical Field

This Invention relates to new antacid products and processes for producing products for treating acidity related aliments in human as well as animals.

### Background of the invention

Acidity related gastro-intestinal disorders include acid refluxes, duodenal ulcers, stomach ulcers and other clinical conditions arising from increase in acidity. These conditions are treated mainly by means of conventional route of add neutralization, the products which rule OTC (Over The Counter) market, or by a new approach of prevention of add secretion based on H₂ receptor antagonists or proton pump inhibitors, which are pharmaceutically active molecules and are prescription medicines.

As per US FDA, to qualify for an OTC composition as an antacid, unit dosage should have minimum of an Acid Neutralization Capacity of 5 milliequivalents. Acid Neutralization Capacity has been defined in "Remington: The Science and Practice of Pharmacy", Vol II, p. 1220, publishers Lippincott Williams & Wilkins, Philadelphia, 20th Edition, 2002 as "the number of milliequivalents of hydrochloric add required to maintain 1 mL of an antacid suspension at pH 3 for 2 hr *in vitro*. This definition when read in terms of "unit dosage" to qualify for an OTC composition as antacid as per US FDA means a dosage that will neutralize 5 milliequivalents or more of hydrochloric acid and maintain its pH to 3 or above in 2 hours *in vitro*.

Acid neutralization is achieved in the Acid Neutralizing Antacid compositions mainly by resorting to use of one or a combination of compounds of alkali metals or alkaline earth metals. Occasionally such compositions are supplemented by use of buffers to make them more effective. These inorganic acid neutralizing salts, although effective in relieving acidity related conditions, have their own side effects, which are worth avoiding if possible, but are inevitable unless viable alternative is found.

Never in the past was it anticipated that an acid neutralizing antacid composition mainly based on proteins and their derivatives shall be a viable alternative.

To explore a total or partial alternative to inorganic antacids totally or partially, work was carried out in this invention on potential and manner in which protein and protein derivatives can be used as antacids. Proteins and their derivatives are useful basically as nutrients. They have very few side effects, if at all, when compared to several side effects of inorganic antacids.

Undesirable side effects arising from inorganic antacids have been described elaborately in literature. A systemic antacid, such as sodium bicarbonate can cause alkalosis and electrolyte imbalances. Most of the antacids are derived from aluminium, calcium and magnesium, which are not so readily soluble. However, they form relatively insoluble compounds in the digestive tract and they are also absorbed systemically leading to toxicity. This absorption may enhance significantly in case of patients heaving ulcerative conditions. ingestion of large amount of calcium can result in a milk-alkall syndrome which involves hypercalcemia, alkalosis and renal failure. Magnesium containing antacids in the long run can cause hyper magnesemia and diarrhea. Aluminium containing antacids in prolonged use can cause phosphate depletion, osteoporosis and osteomalacia as well as neurotoxicity. All the toxicities of aluminium, calcium- and magnesium-containing antacids are common and more pertinent in patients with renal failure.

### Summary of Invention

This invention provides a pharmaceutical dosage composition for oral consumption by mammals including a human being for use in treating gastric acidity related disorders, the composition comprising a protein present in an amount sufficient to contribute fully to the antacid efficacy: of the composition on its own, the presence of other active antacid agents in the composition being optional, wherein the protein comprise:
a) a leaf protein concentrate or isolate partly or fully isolated from its natural source;
   and wherein the composition may further optionally comprise:
b) one or more of pharmaceutically permitted additives, colors, antioxidants, vitamins, minerals, preservatives, buffers, excipients, flavors, sweeteners;
c) other active pharmaceutical ingredients.

The invention also provides use of a protein in the preparation of a pharmaceutical, dosage composition for oral consumption by mammals including a human being for treating gastric acidity related disorders, wherein the protein is present in an amount sufficient to contribute fully to the antacid efficacy of the said composition on its own, the presence of other active antacid agents in the composition being optional, wherein the protein comprises:
a) a leaf protein concentrate or isolate partly or fully isolated from its natural source;
   and wherein the composition may further optionally comprise:
b) one or more of pharmaceutically permitted additives, colors, antioxidants, vitamins, minerals, preservatives, buffers, excipients, flavors, sweeteners;
c) other active pharmaceutical ingredients.

### Detailed description of invention

This invention originated from a surprising observation that usual symptoms of hyperacidity, which included post meal burning sensation in the stomach, moderate headache, moderate backache etc. were relieved reproducibly within a minute Whenever a wet, fiber free and water solubles free nutrient concentrate preparation, Leaf Protein Concentrate (LPC), extracted from leaves of fenugreek (Trigonella foenum-graecum L), a common green leafy vegetable (*Methi* in vernacular local language Marathl) was eaten even in as small quantity as less than 1 gram on dry basis.

Proteins and compositions rich in proteins in native form show very little acid neutralizing capacity in the form as they occur in the nature. It is perhaps for this reason that they were never anticipated as acid neutralizing agents. Yet they are useful as antacid agents. It is true that normal diet does have proteins as normal part of the diet and must be serving antacid functions when hydrolysed in stomach. Yet the idea of using protein concentrates as antacid agents is not the same as use of food proteins as part of food for antacid effects.

In hyperacidity conditions, what is required is management of more than normal secretion of acids over and above capability of protein component of normal diet to deal with them. Proteins form a small percentage of normal diet components, and for intake of an extra amount of protein to serve the extra acidity neutralization requirement, if aimed to be met through normal diet components, will require impractical increase in the amount of normal dietary components that will have to be eaten and that too accompanied by unacceptable extra intake of calories. Further limitation is that proteins eaten as part of natural dietary components need not be all available for digestion and hydrolysis in the time they reside in stomach.

It is, however feasible to achieve extra intake of proteins, if isolates and concentrates of proteins having excellent Biological Value (Percentage of nitrogen retained in the body of the percentage of nitrogen absorbed in the digestive tract) extracted from their natural sources could be supplemented. This could be a first step in managing mild hyperacidity conditions and where prolonged control and slower onset of acid neutralization is desired. In addition to antacid effect, this protein supplementation shall serve beneficial nutritional purpose too, rather than side effects which inorganic antacids exhibit.

In this invention, it is seen that although in native form the buffering power of proteins is weak from chemical point of view, they are actually delayed release buffers. They are polymers of bipolar molecules with amino as well as carboxyl groups. Their real buffering power is bound in the form of peptide bonds which start getting released by peptic hydrolysis as soon as the protein reaches the stomach. This slow release of antacid activity is a feature which makes proteins far more superior to inorganic alkaline compounds as antacids, particularly because high alkalinity of inorganic alkaline antacids after acid neutralization results in increase in stomach alkalinity which results into an "acid rebound" i.e. stimulation of stomach to secrete more acid due to alkaline pH in the stomach.

In one embodiment of this invention, the acid neutralizing agent can be a protein concentrate or protein isolate. Protein concentrate or isolate are compositions of proteins usually prepared from natural sources by a process which results in products richer in protein than the original raw material. Being extracted from their natural raw materials, they are free from non-protein components and fully exposed to enzymatic action *in vitro* as well as when ingested in the stomach and the released hydrolysate shall impart its buffering effect in control of pH.

When more severe hyperacidity conditions are required to be handled and rapid release of already available antacid activity is desired, it would be more worthwhile to include in that embodiment of antacid treatment compositions, the peptic digests of the said isolates and concentrates of good Biological Value proteins or well balanced mixtures of amino acids derived from high Biological Value proteins. The digests could be produced in several different ways including using papain or any other proteolytic enzyme or could be an alkaline or acid digest done and protein degradation in such mixtures could have been up to various levels of degradation through a mixture of large polypetides though oligo peptides, tri- and di-peptides to amino add level. Individual amino acids produced synthetically or by fermentation route may also be used directly in this case. These embodiments give effective acid neutralization incapacity between pH 6.5 to pH 3.

Enzymatic protein digests may also be subjected to "plastein reaction", a reversal of hydrolysis catalysed by protelolytic enzymes under high unusually high substrate (a hydrolysate generated by proteotytic enzyme) concentration, of 50% or more, which generates products called "plasteins", which are also included as antacid active included in this invention. Plastein reaction Is resorted to for achieving various objectives such as removal of bitterness and undesirable flavors (Fujimaki, M., Kato, H., Arai, S., and Tamaki, E. 1968. Food Technology, 22, page no. 889), for generation of agreeable flavors (Savangikar V.A and Joshi, R.N. 1979. J.Sci.food:Agric., 30, page no. 899), for generating therapeutic nutritional protein supplements (Yamashita, M., Arai, S., Tsai. S. J. and Fujimaki, M. 1971. J. Agr. Food Chem., 19, page no. 1151).

To handle still more severe conditions of hyperacidity, acid neutralization capacity of the untreated proteins and their hydrolysed derivatives including amino acids and sodium salts of amino acids and equivalent compounds can be improved markedly by making their alkali derivative, which are very useful for preparing antacid compositions.

The alkali treatment is possible in cold as well as hot condition and the alkali derivative formed having an alkaline pH are novel products. Sodium hydroxide treatment is most convenient for this purpose, although other alkalles may also be used and are covered in this invention. This results in an alkaline product which can be made free from un-reacted alkali by titrating extra alkall above pH 10.8 by acid lor by dialysis and such a dialysed product having alkaline pH (between pH 8 to 10.8) has further enhanced Acid Neutralization Capacity which sets rapidly as soon as ingested. Such an alkali derivative when it comes in contact with acids in stomach, shall return to its pre-treatment form, which is nothing but a normal nutrient in the form of native protein or peptides or amino acids having a beneficial effect and practically no or fewer side effects if at all, when compared to inorganic antacids. Far less quantity of such an alkali derivative will be needed to neutralize same quantity of acids in stomach and to comply with minimum requirements to qualify as antacid composition, such as the US FDA recommendations of minimum Acid Neutralization Capacity of 5 per dose.

Antacid effect may be available even from use of a single amino acid or its derivatives such as glutamic acid and sodium glutamate.

However, this avenue is inferior to use of dietary proteins for this effect since its beneficial effect as nutrient besides antacid effect would be available only if the amino acid is in a balanced amount with respect to other essential amino acids in the same diet, so that the absorbed amino acids are assimilated for tissue build up and repair and not excreted unassimilated or unutilized. This aspect brings in the focus preference that should be given to high Biological Value dietary proteins and their derivatives and balanced mixtures of essential (those amino acids which are not synthesized in animal body and must be supplied through food for normal protein metabolism) amino acids, which are characterized by high retention / assimilation inside the body after they are absorbed in the digestive tract, when use for the purpose of antacid application is the objective.

In normal circumstances, the additional protein that shall be ingested in antacid compositions is not significant enough to bother for balancing against other dietary protein sources. However, when protein intake is a limitation for a patient, such as in renal failures, it is possible to balance this extra input through isolates and concentrates of proteins by withdrawing appropriate quantity of protein contributing components of conventional diet including but not limited to pulses, meat, groundnut etc.

As a matter of course, basically this invention is valuable in usual hyperacidity patients to relieve them of side effects of inorganic antacids. However, over and above this, it is also valuable to most of the patients who are undergoing treatment on various other diseases who are temporarily vulnerable to acidity caused by intake of medicines prescribed to them. Inclusion of antacids based on proteins, their derivatives and alkaline derivatives shall be a very valuable way of relieving them from the side effects of acidity generated by medicine treatment to them on other disease conditions. Incidentally, most of the medicines that need to be ingested are known to cause hyperacidity. Thus, in most of the disease treatments, use of antacids of this invention either with the therapy or incorporated in the unit dosage of the medicine may be valuable as a normal course of treatment.

In practical course, it may be necessary to use two or more forms of the antacid actives covered in this invention to cover broad range of antacid protection desired such as immediate onset as well as up to few hours after the meals.

In above assortment of protein and related products available, It is possible to visualize antacid preparations comprising proteins or their derivatives with or without alkaline treatment with total absence of alkali metal or alkaline earth metal compounds in such compositions, or with various proportions of the same in small quantity and also with or without buffers such as Potassium Hydrogen Phosphate.

The active ingredient, the isolates and concentrates of proteins, their hydrolysed derivatives and alkali derivatives of proteins and their hydrolysed products and amino acids, may be delivered to the patient through various solid as well as liquid dosage forms normally known in the art of pharmaceuticals including but not limited to powder dosage forms effervescent or otherwise, compacted forms such as granules and tablets of various forms and shapes, layered tablets, fast and slow release tablets, incorporated In capsules of slow release type or otherwise, syrups etc. the antacid active Ingredient of this invention may also be incorporated in therapeutic food formulations for general healthcare, in a beverage or specific treatment including deriving an antacid effect, or with other pharmaceutically active ingredients In various dosage forms known in the art. This invention covers all pharmaceutical compositions described above and their analogous products obvious to a person in the field of art of pharmaceuticals and antacids antacids. Proteins, their hydrolysates including individual amino acids and alkali derivatives of proteins and their hydrolysates and individual amino acids and the said compositions may contain one or more of other pharmaceutically acceptable Excipients, colors. flavors, binders, conventional as well as high intensity low calorie sweeteners including but not limited to saccharine, aspartem. acesulphame K, trichlorogalactosucrose (sucralose), stevioside, maltitol, lactitol, fructose, high fructose com syrup, honey.

The said antacid ingredients of this invention including proteins, their hydrolysed and other derivatives are of natural origin extracted from plants.

The description and examples, conditions of treatment provided here are for the purpose of illustrating the invention and any modification and improvements obvious to persons skilled in the art are also included in this disclosure of the claimed invention.

Dietary protein concentrates, isolates, their hydrolysates and their derivatives are as embodiments of this invention.

Of the several embodiments possible within the scope of the disclosure made here, some representative embodiments are mentioned below. The scope of the patent is not limited by the embodiments mentioned and any other embodiments within the scope of this invention are included herein.

Examples given below are illustrative for the purpose of explaining how the invention is to be performed.

Some of the examples have been included for reference purpose only and do not fall under the scope of the claims.

The examples given below illustrate the invention further. Active ingredients use, range of reaction conditions and range of reactants and other techniques used in the experiments are only illustrative and in no way limit the scope of the invention. Any variation obvious to a person skilled in the art and necessary for the purpose of scale up to commercial scale and optimal operation is included in the invention claimed here.

The proteins and related compounds described as antacid actives in above specification for the treatment of acidity and related disorders may be given in the form of a nutritional supplement or as a medication or both or given as a part of beverages too.

### EXAMPLE: 1

### MATERIALS & METHODS

### 1) PROTEIN RICH PREPARATIONS USEFUL IN ANTACID PREPARATIONS

Casein Hammerstein (isolate of milk protein) available from BDH Chemicals was used as one example of protein isolate from milk for illustrating this invention.

Protein concentrate extracted from juice of plant shoots (Leaf Protein Concentrate, also known as LPC) in native extracted form as water insoluble coagulum and in the form of its peptic hydrolysate is used to give an example from plant source as well as of use of a hydrolysed protein.

Several methods of preparation of LPC exist, which are covered and reviewed comprehensively by Pirie, 1971 (Pirie, N.W., 1971, IBP Handbook no. 20, Blackwell, Oxford). In the method used here, the protein concentrate is a heat precipitated (80 degrees celcius) coagulum of the juice expressed from pulp of the shoots of *Methi* (Trigonella foenum-graecum L.), a commonly eaten leafy vegetable in India. The pulp was made in a domestic kitchen mixer. The coagulum was washed several times in water, pressed free from excess water through cotton cloth, mixed with about 30% by weight of common salt and stored in refrigerator until used. Composition of such LPC, on dry weight basis, ranges between crude protein (Nitrogen multiplied by 6) 45 to 55%. Crude fat (Ether Extract) 10 to 15%, crude fiber less than 1%, Total ash around 1 to around 6% and acid insoluble ash of about 1%.

The wet salt preserved LPC, immediately before use, was suspended in excess of water, allowed to settle and supernatant water decanted and discarded. The process was repeated until the water wash was free from salty taste. Finally, it was filtered through double layer cotton cloth, washed twice with water, hand squeezed free of excess of water and the wet coagulum was taken for use.

Dry matter content of wet cake differed from batch to batch from between 20 to 37%.

The said LPC can be produced from any other leafy plant species and in a variety of different methods of production and any product analogous to LPC produced by any process is covered in the embodiment of this invention for the purpose of application in antacid preparations. LPC is a very high Biological Value dietary protein. It has been subjected to several human feeding trials including children feeding trials and has been described as safe for human consumption. Pirie, 1971 referred above has covered a comprehensive information on LPC. Load of nitrogen and degradation products of protein metabolism that has to be disposed off through kidneys is, incidentally far less when high Biological Value protein is ingested than when a protein of low Biological Value is ingested.

### 2) PREPARATION OF PROTEIN HYDROLYSATE - PEPTIC (ENZYMATIC)

Peptic digest of LPC was prepared in 0.1 N HCl by using pepsin (1:10,000) available from Difco. The digest was adjusted to pH 5.6, filtered through cotton cloth to get rid of undigested LPC, the filtrate concentrated and diluted appropriately by distilled water when required to be used.

Several methods are known in the art for enzymatic proteolysis. All these variations are included in the embodiment of this invention and may be followed for deriving hydrolysate for this invention.

### 3) PREPARATION OF ALKALI TRETAED ANTACID PRODUCT FROM ACTIVE INGREDIENTS SUCH AS PROTEIN, PROTEIN HYDROLYSATE OR AMINO ACIDS

About 20 ml of 1N NaOH was added to about or less than 1 gram dry weight of the active ingredient (Protein isolate or protein rich extract) with about 10 ml water content. In cold treatment, the mixture was kept at room temperature overnight until the sedimented coagulum was seen to have been dispersed evenly in a solution. In a hot treatment, the mixture was kept in an oven at 80 degrees celcius, volume restored to 30 ml by addition of distilled water and titrated after various stages of degradation after the period of treatment. Alkali remaining in excess was either neutralized by titrating against 1N HCl until pH dropped to 10.8 or by dialyzing the mixture using gelatin membrane against 700 milliliter portions of distilled water three to four times until the dialysate contained little alkalinity.

The alkali treatment may, optionally, be terminated immediately after the addition of alkali, or continued for desired number of hours or days depending up on the degree of alkalinity of end product desired..

### DETERMINATION OF ACID NEUTRALIZATION CAPACITY (ANC) OF PROTEINS AND THEIR DERIVATIVES:

Acid Neutralization Capacity was noted as milliequivalents of HCl neutralized to maintain the pH at 3 for two hours and expressed as per gram dry weight of the active antacid ingredient or per dose of the antacid composition containing the active ingredient. Total volume of the reactants were adjusted to 30 ml or 40 ml and titrated against 1 N HCl and pH was noted after each small dropwise addition. The pH was measured by a pH meter.

In titration of distilled water, it was observed that pH of the water raised rapidly to 10.8 with addition of a single drop. Similarly, while titrating 1 N NaOH against 1N HCl, fall in pH from 12.2 to 10.8 was gradual. However, thereafter, just one drop was sufficient to lower the pH to neutral. Thus, it was clear that acid neutralized by the products within the pH range of 3 to 10.8 was due to Acid Neutralization Capacity when solutions of antacid active ingredients covered in the examples were titrated for determination of their ANC.

When alkali treated mixtures were titrated, acid required to bring down the pH to 10.8 was considered as acid required to neutralize the unreacted NaOH. Acid required to neutralize pH from 10.8 up to pH 3 was taken for calculation of ANC.

### DIALYSIS

Whenever desired, the alkali treated mixture was dialysed through gelatin membrane against about 700 milliliters of distilled water each time three to four times. Significant quantity of alkalinity appeared in first two dialyses in first two hours. Third dialysis, usually done for several hours overnight usually contained very little of alkalinity in the range of pH 10.8 and above.

### EXAMPLE 2

### ACID NEUTRALIZATION CAPACITY OF ACTIVE INGREDIENTS

Following are the results of ANC of various antacid actives prepared and tested by methods given above:

Protein isolate of untreated Casein had ANC of 0.12 per gram.

The LPC showed ANC of 0.44 per gram dry matter. ANC of peptic digest of LPC was found to be 1.53 per gram dry matter. Thus, the actual ANC available from LPC when ingested even in its native form is not 0.24 gram per dry matter, but 1.53 gram per gram dry matter.

ANC of 1.5 per gram dry matter is practically relevant because a dose of about 4 gram at one time is practically feasible to be administered orally through tablets, effervescent powder formulations or flavored syrups, to enable delivery of a minimum ANC of 5 gram per dose.

Cold alkali treatment raised ANC of casein to 1.5. Hot alkali treatment of 2 days raised ANC to 4.88 and of 6 days to 8.

Cold alkali treatment raised ANC of LPC to 2.75. Hot alkali treatment of 4 hours raised ANC to 3.6, of one day to 5.5 and of 6 days to 7.9.

The un-reacted alkali in the alkali treated proteins can be either eliminated by titration against acid to lower the pH to 10.8. Dialysis of the alkali treated mixture through gelatin membrane against distilled water resulted in salt free and NaOH free alkaline product which ranged from pH of 10.6 to 8 depending on how much of low molecular weight component was lost in the water against which dialysis was made (wash water). When acid neutralized by the wash water below pH 10.8 and dry matter content of such washes (as corrected for weight of NaCl formed by NaOH neutralized above pH 10.8) was combined with the acid neutralized by the retaintate (solution retained inside the dialysis membrane) and dry matter content of the retaintate, it was seen that the combined ANC was around 6 for LPC in 4 hour treatment of hot alkali.

It is clear that alkali derivatives, cold as well as hot treated, of the proteins, their derivatives and amino acids are feasible antacid agents to give practical compositions with other ingredients to provide acid neutralization capacity of 5 or above. The untreated LPC shall still be useful as delayed release active because peptic digest of the same showed ANC of 1.5 between pH 5.6 to 3 and this capacity shall get reflected when digested in stomach. Same can be said to happen for casein too. The actual quantity of the actives of this invention used in an antacid composition will depend upon the local rules governing ANC of antacid preparations and requirements of the treatment of the disease indication aimed for alleviation.

### EXAMPLE 3

### AMINO ACIDS AND THEIR ALKALINE DERIVATIVE:

About one milliequivalent of NaOH was neutralized per milliequivalent of Glutamic acid in water to raise the pH of its aqueous solution from 2.9 to about 5. Thereafter, there was rapid increase in pH to 8.4 followed by slower increase in pH and from pH 5 to 10.9, about 1.2 milliequivalents of NaOH was consumed per milliequivalent of glutamic acid used. The product upto pH 5 is analogous to sodium glutamate formation, which in itself is analogous to an intermediate form of analogous protein derivative. A product formed from the amino acid as well as protein above the range of pH 5 was novel and provided for substantially extra ANC.

### EXAMPLE 4

### SODIUM GLUTAMATE AND ITS ALKALINE DERIVATIVE

Crystalline Sodium Glutamate gave solution of pH 6.7 It took, per millliequivalent of Sodium Glutamate used, little less than 1 milliequivalent of HCl to bring the pH from 6.7 down to 3 and about 1 milliequivalent of HCl to raise the pH from 6.7 to 10.8. Here, the crystalline Sodium Glutamate solution of pH 6.7 represents an intermediate form of alkaline protein. The form of Sodium Glutamate formed above pH 6.7 was novel and analogous to the alkaline form of protein in that pH range and both are novel products providing for substantially improved ANC.

## Claims

1. A pharmaceutical dosage composition for orale consumption by mammals including a human being for use in treating gastric acidity related disorders, the composition comprising a protein present in an amount sufficient to contribute fully to the antacid efficacy of the composition on its own, the presence of other active antacid agents in the composition being optional, wherein the protein comprises:
a) a leaf protein concentrate or isolate partly or fully isolated from its natural source;
and wherein the composition may further optionally comprise:
b) one or more of pharmaceutically permitted additives, colors, antioxidants, vitamins, minerals, preservatives, buffers, excipients, flavors, sweeteners;
c) other active pharmaceutical ingredients.

2. A composition according to claim 1 wherein said optional other active pharmaceutical ingredients and other antacid agents include: one or more of antibiotics, analgesics, anti-pyretics, cardio-vascular drugs, anti-inflammatory drugs, muscle relaxants, H₂ receptor antagonists, proton pump inhibitors, drugs that enhance stomach mucosal protection, prokinetic drugs that enhance gastrointestinal motility, digestants, pancreatic enzymes and laxatives.

3. A composition according to claim 1 wherein the pharmaceutical dosage composition is in one or more of solid, liquid or gel dosage forms including tablets, granules, powder, effervescent as well as non-effervescent forms, rapid release or slow release forms, encapsulated forms, syrups.

4. A composition according to claim 1, wherein the pharmaceutical dosage composition is obtainable by a process comprising:
a) taking the said protein;
b) optionally adding thereto: one or more of other inorganic antacids including Sodium Bicarbonate, Aluminium Hydroxide, Bismuth Salicylate, Calcium Carbonate, Dihydroxyaluminium Sodium Carbonate, Magnesium Hydroxide, Magnesium Oxide, Magnesium Trisilicate, Sodium Carbonate and mixtures thereof; buffers permitted in food including sodium acetate, Dipotassium hydrogen phosphate, potassium dihydrogen phosphate; a H2-Receptor antagonist in pharmaceutically effective quantity;
c) optionally adding thereto one or more of pharmaceutically acceptable flavouring, excipients, coloring matters, additives, extenders, sweeteners, effervescence generating ingredients.

5. Use of a protein in the preparation of a pharmaceutical dosage composition for oral consumption by mammals including a human being for treating gastric acidity related disorders, wherein the protein is present in an amount sufficient to contribute fully to the antacid efficacy of the said composition on its own, the presence of other active antacid agents in the composition being optional, wherein the protein comprises:
a) a leaf protein concentrate or isolate partly or fully isolated from its natural source;
and wherein the composition may further optionally comprise:
b) one or more of pharmaceutically permitted additives, colors, antioxidants, vitamins, minerals, preservatives, buffers, excipients, flavors, sweeteners;
c) other active pharmaceutical ingredients.

6. Use according to claim 5 wherein said optional other active pharmaceutical ingredients and other antacid agents include one or more of antibiotics, analgesics, anti-pyretics, cardio-vascular drugs, anti-inflammatory drugs, muscle relaxants, H₂ receptor antagonists, Proton pump inhibitors, drugs that enhance stomach mucosal protection, prokinetic drugs that enhance gastrointestinal motility, digestants, pancreatic enzymes and laxatives.

7. Use according to claim 5 wherein the pharmaceutical dosage composition is in one or more of solid, liquid or gel dosage forms including tablets, granules, powders, effervescent as well as non-effervescent forms, rapid release or slow release forms, encapsulated forms, syrups.

8. Use according to claim 5, wherein the pharmaceutical dosage composition is obtainable by a process comprising:
a) taking the said protein;
b) optionally adding thereto one or more of other inorganic antacids including Sodium Bicarbonate, Aluminium Hydroxide, Bismuth Salicylate, Calcium Carbonate, Dihydroxyaluminium Sodium Carbonate, Magnesium Hydroxide, Magnesium Oxide, Magnesium Trisilicate, Sodium Carbonate and mixtures thereof; buffers permitted in food including sodium acetate, Dipotassium hydrogen phosphate, potassium dihydrogen phosphate; a H2-Receptor antagonist in pharmaceutically effective quantity;
c) optionally adding thereto one or more of pharmaceutically acceptable flavoring, excipients, coloring matters, additives, extenders, sweeteners, effervescence generating ingredients.

## Patentansprüche

1. Eine pharmazeutische Dosierungszusammensetzung zur oralen Einnahme durch Säuger, einschließlich Menschen, zur Verwendung bei der Behandlung von magensäurebedingten Erkrankungen, wobei die Zusammensetzung ein Protein umfasst, das in einer ausreichenden Menge vorhanden ist, um alleine in vollem Umfang zur säurebindenden Wirksamkeit der Zusammensetzung beizutragen, und wobei das Vorhandensein sonstiger säurebindender Wirkstoffe in der Zusammensetzung fakultativ ist, worin das Protein Folgendes umfasst:
a) ein Blattproteinkonzentrat oder -isolat, das teilweise oder vollständig aus seinem natürlichen Ausgangsmaterial isoliert wurde;
und worin die Zusammensetzung überdies wahlweise Folgendes beinhalten kann:
b) einen oder mehrere pharmazeutisch zulässige Zusatzstoffe, Farbstoffe, Antioxidantien, Vitamine, Mineralstoffe, Konservierungsstoffe, Puffer, Hilfsstoffe, Geschmackstoffe, Süßstoffe;
c) sonstige aktive pharmazeutische Inhaltsstoffe.

2. Eine Zusammensetzung gemäß Anspruch 1, worin die besagten fakultativen sonstigen aktiven pharmazeutischen Inhaltsstoffe und sonstigen säurebindenden Wirkstoffe eines oder mehrere der folgenden Mittel umfassen: Antibiotika, Analgetika, fiebersenkende Mittel, Herz-Kreislauf-Mittel, Entzündungshemmer, Muskelrelaxantien, H₂-Rezeptor-Antagonisten, Protonenpumpenhemmer, Arzneimittel zum verbesserten Schutz der Magenschleimhaut, prokinetische Arzneimittel zur Verbesserung der gastrointestinalen Motilität, verdauungsfördernde Mittel, Pankreasenzyme und Laxantien.

3. Eine Zusammensetzung gemäß Anspruch 1, worin die pharmazeutische Dosierungszusammensetzung sich in einer oder mehreren festen, flüssigen oder gelförmigen Darreichungsformen befindet, etwa Tabletten, Granulate, Pulver, Brausesowie nicht Brauseformen, Arzneiformen mit schneller oder langsamer Wirkstofffreisetzung, Kapselformen, Sirupe.

4. Eine Zusammensetzung gemäß Anspruch 1, worin die pharmazeutische Dosierungszusammensetzung durch ein Verfahren erhältlich ist, das Folgendes umfasst:
a) Gewinnen des besagten Proteins;
b) wahlweise Hinzufügen eines oder mehrerer sonstiger anorganischer Antazida, etwa Natriumbicarbonat, Aluminiumhydroxid, Bismutsalicylat, Calciumcarbonat, Dihydroxyaluminiumnatriumcarbonat, Magnesiumhydroxid, Magnesiumoxid, Magnesiumtrisilicat, Natriumcarbonat und Mischungen davon; von in Nahrungsmitteln erlaubten Puffern wie Natriumacetat, Dikaliumphosphat, Kaliumdihydrogenphosphat; eines H2-Rezeptor-Antagonisten in einer pharmazeutisch wirksamen Menge;
c) wahlweise Hinzufügen eines oder mehrerer pharmazeutisch akzeptabler Geschmackstoffe, Hilfsstoffe, Farbstoffe, Zusatzstoffe, Streckmittel, Süßstoffe sowie Inhaltsstoffe, die ein Sprudeln verursachen.

5. Verwendung eines Proteins bei der Herstellung einer pharmazeutischen Dosierungszusammensetzung zur oralen Einnahme durch Säuger, einschließlich Menschen, zur Behandlung von magensäurebedingten Erkrankungen, worin das Protein in einer ausreichenden Menge vorhanden ist, um alleine in vollem Umfang zur säurebindenden Wirksamkeit der besagten Zusammensetzung beizutragen, wobei das Vorhandensein sonstiger säurebindender Wirkstoffe in der Zusammensetzung fakultativ ist, worin das Protein Folgendes umfasst:
a) ein Blattproteinkonzentrat oder -isolat, das teilweise oder vollständig von seiner natürlichen Ausgangsmaterial isoliert wurde;
und worin die Zusammensetzung überdies wahlweise Folgendes beinhalten kann:
b) einen oder mehrere pharmazeutisch zulässige Zusatzstoffe, Farbstoffe, Antioxidantien, Vitamine, Mineralstoffe, Konservierungsstoffe, Puffer, Hilfsstoffe, Geschmackstoffe, Süßstoffe;
c) sonstige aktive pharmazeutische Inhaltsstoffe.

6. Verwendung gemäß Anspruch 5, worin die besagten fakultativen sonstigen aktiven pharmazeutischen Inhaltsstoffe und sonstigen säurebindenden Wirkstoffe eines oder mehrere der folgenden Mittel umfassen: Antibiotika, Analgetika, fiebersenkende Mittel, Herz-Kreislauf-Mittel, Entzündungshemmer, Muskelrelaxantien, H₂-Rezeptor-Antagonisten, Protonenpumpenhemmer, Arzneimittel zum verbesserten Schutz der Magenschleimhaut, prokinetische Arzneimittel zur Verbesserung der gastrointestinalen Motilität, verdauungsfördernde Mittel, Pankreasenzyme und Laxantien.

7. Verwendung gemäß Anspruch 5, worin die pharmazeutische Dosierungszusammensetzung sich in einer oder mehreren festen, flüssigen oder gelförmigen Darreichungsformen befindet, etwa Tabletten, Granulate, Pulver, Brausesowie nicht Brauseformen, Arzneiformen mit schneller oder langsamer Wirkstofffreisetzung, Kapselformen oder Sirupe.

8. Verwendung gemäß Anspruch 5, worin die pharmazeutische Dosierungszusammensetzung durch ein Verfahren erhältlich ist, das Folgendes umfasst:
a) Gewinnen des besagten Proteins;
b) wahlweise Hinzufügen eines oder mehrerer sonstiger anorganischer Antazida, etwa Natriumbicarbonat, Aluminiumhydroxid, Bismutsalicylat, Calciumcarbonat, Dihydroxyaluminiumnatriumcarbonat, Magnesiumhydroxid, Magnesiumoxid, Magnesiumtrisilicat, Natriumcarbonat und Mischungen davon; von in Nahrungsmitteln erlaubten Puffern wie Natriumacetat, Dikaliumphosphat, Kaliumdihydrogenphosphat; eines H2-Rezeptor-Antagonisten in einer pharmazeutisch wirksamen Menge;
c) wahlweise Hinzufügen eines oder mehrerer pharmazeutisch akzeptabler Geschmackstoffe, Hilfsstoffe, Farbstoffe, Zusatzstoffe, Streckmittel, Süßstoffe sowie Inhaltsstoffe, die ein Sprudeln verursachen.

## Revendications

1. Composition pharmaceutique à administration par voie orale destinée aux mammifères dont l'être humain pour l'utilisation dans le traitement des troubles liés à l'acidité gastrique, la composition contenant une protéine présente en quantité suffisante pour contribuer pleinement à l'efficacité antiacide de la composition seule, la présence d'autres agents antiacides actifs dans la composition étant optionnelle, la protéine contenue dans la composition comprenant :
a) un concentrat ou un isolat de protéine de feuilles, isolé partiellement ou entièrement de sa source naturelle ;
et ladite composition pouvant aussi comprendre optionnellement :
b) un ou plusieurs additifs, colorants, antioxydants, vitamines, minéraux, conservateurs, tampons, excipients, arômes, édulcorants, dont l'utilisation pharmaceutique est autorisée ;
c) d'autres substances pharmaceutiques actives.

2. Composition selon la revendication 1, dans ladite composition lesdites autres substances pharmaceutiques actives et lesdits autres agents antiacides pouvant être inclus(es) en option comprenant un ou plusieurs antibiotiques, analgésiques, antipyrétiques, médicaments cardiovasculaires, médicaments anti-inflammatoires, relaxants musculaires, antagonistes des récepteurs H₂, inhibiteurs de la pompe à protons, médicaments stimulant la protection des muqueuses stomacales, médicaments procinétiques stimulant la motilité gastrique, médicaments digestifs, enzymes pancréatiques et laxatifs.

3. Composition selon la revendication 1, ladite composition pharmaceutique pouvant être sous une ou sous plusieurs formes de type solide, liquide ou gel, comprenant les comprimés, les granulés et les poudres, effervescent(e)s ou non effervescent(e)s, à libération rapide ou à libération lente, les formes encapsulées et les sirops.

4. Composition selon la revendication 1, ladite composition pharmaceutique étant obtenue par la voie d'un procédé comprenant :
a) l'extraction de ladite protéine ;
b) optionnellement l'addition à celle-ci d'un ou de plusieurs autres antiacides inorganiques de type bicarbonate de sodium, hydroxyde d'aluminium, salicylate de bismuth, carbonate de calcium, carbonate sodique de dihydroxyaluminium, hydroxyde de magnésium, oxyde de magnésium, trisilicate de magnésium, carbonate de sodium et des mélanges de ceux-ci ; tampons, dont l'utilisation alimentaire est autorisée, de type acétate de sodium, hydrogénophosphate de potassium, dihydrogénophosphate de potassium ; d'un antagoniste des récepteurs H₂, en quantité pharmaceutiquement efficace ;
c) optionnellement l'addition à celle-ci d'un ou de plusieurs aromatisants, excipients, colorants, additifs, produits de charge, édulcorants, éléments produisant l'effervescence, dont l'utilisation pharmaceutique est autorisée.

5. Utilisation d'une protéine dans la préparation d'une composition pharmaceutique à administration par voie orale destinée aux mammifères dont l'être humain pour l'utilisation dans le traitement des troubles liés à l'acidité gastrique, ladite protéine étant présente en quantité suffisante pour contribuer pleinement à l'efficacité antiacide de ladite composition seule, la présence d'autres agents antiacides actifs dans la composition étant optionnelle, ladite protéine comprenant :
a) un concentrat ou un isolat de protéine de feuilles, isolé partiellement ou entièrement de sa source naturelle ;
et ladite composition pouvant aussi comprendre optionnellement :
b) un ou plusieurs additifs, colorants, antioxydants, vitamines, minéraux, conservateurs, tampons, excipients, arômes, édulcorants, dont l'utilisation pharmaceutique est autorisée ;
c) d'autres substances pharmaceutiques actives.

6. Utilisation selon la revendication 5, lesdites autres substances pharmaceutiques actives et lesdits autres agents antiacides pouvant être inclus(es) en option comprenant un ou plusieurs antibiotiques, analgésiques, antipyrétiques, médicaments cardiovasculaires, médicaments anti-inflammatoires, relaxants musculaires, antagonistes des récepteurs H₂, inhibiteurs de la pompe à protons, médicaments stimulant la protection des muqueuses stomacales, médicaments procinétiques stimulant la motilité gastrique, médicaments digestifs, enzymes pancréatiques et laxatifs.

7. Utilisation selon la revendication 5, ladite composition pharmaceutique pouvant être sous une ou sous plusieurs formes de type solide, liquide ou gel, comprenant les comprimés, les granulés et les poudres, effervescent(e)s ou non effervescent(e)s, à libération rapide ou à libération lente, les formes encapsulées et les sirops.

8. Utilisation selon la revendication 5, ladite composition pharmaceutique étant obtenue par la voie d'un procédé comprenant :
a) l'extraction de ladite protéine ;
b) optionnellement l'addition à celle-ci d'un ou de plusieurs autres antiacides inorganiques de type bicarbonate de sodium, hydroxyde d'aluminium, salicylate de bismuth, carbonate de calcium, carbonate sodique de dihydroxyaluminium, hydroxyde de magnésium, oxyde de magnésium, trisilicate de magnésium, carbonate de sodium et des mélanges de ceux-ci ; tampons, dont l'utilisation alimentaire est autorisée, de type acétate de sodium, hydrogénophosphate de potassium, dihydrogénophosphate de potassium ; d'un antagoniste des récepteurs H₂, en quantité pharmaceutiquement efficace ;
c) optionnellement l'addition à celle-ci d'un ou de plusieurs aromatisants, excipients, colorants, additifs, produits de charge, édulcorants, éléments produisant l'effervescence, dont l'utilisation pharmaceutique est autorisée.
